# EUROPEAN PATENT SPECIFICATION

(11) **EP 2 280 738 B1**
(45) Date of publication and mention of the grant of the patent: **16.05.2012**
(21) Application number: 09726401.4
(22) Date of filing: 27.03.2009
(51) Int. Cl.: A61L 27/18

(54) **A VERTEBROPLASTY METHOD USING AN ADDITION CURABLE POLYSILOXANE SYSTEM**
VERTEBROPLASTIE-VERFAHREN MIT EINEM ADDITIONSHÄRTBAREN POLYSILOXAN-SYSEM
PROCÉDÉ DE VERTÉBROPLASTIE UTILISANT UN SYSTÈME DE POLYSILOXANE DURCISSABLE PAR ADDITION

(30) Priority: 27.03.2008 US 40007
(43) Date of publication of application: 09.02.2011
(73) Proprietor: Broockeville Corporation N.V., 6545 CH Nijmegen (NL)
(72) Inventor: de VRIES, Jan Albert, 7021 HH Zelhem (NL); HANNES, Raymond Jozef, 3056 LD Rotterdam (NL)
(74) Representative: Volmer, Johannes Cornelis
(86) International application number: PCT/EP2009/002428
(87) International publication number: WO 2009/118203

(56) References cited:
- WO-A1-00/48553
- WO-A2-2007/062082
- US-A1- 2004 254 320
- US-A1- 2005 209 695
- US-A1- 2008 086 143

## Description

### TECHNICAL FIELD

The present invention relates generally to treatments of disordered bony structures, in particular to vertebroplasty compositions and methods and, more specifically, to treating vertebral bodies in living creatures, in particular, human beings and also compositions suitable for use in such methods and preparation and use of such compositions.

### BACKGROUND OF THE ART

Percutaneous vertebroplasty comprises injection of a bone filler composition into a vertebral body to be treated of the spinal column via a percutaneous route. A flowable form of the bone filler composition such as a paste or semi-liquid is injected via a needle of a suitable injection device into the respective vertebral body. Advantageously, the viscosity of the bone filler is such that it also flows into fractures in the vertebral body, if present. The purposes of vertebroplasty include reinforcement of the vertebral body treated and improvement of the compressive strength thereof. The bone filler is injected as a paste or semi-liquid from a suitable gun or injection system via a needle that has been passed into the vertebral body. Vertebroplasty is usually applied to patients that suffer from osteoporotic fractures, malignant metastatic disease, and benign tumors of the bone. Typically the injection is performed under. The bone filler composition commonly used for vertebroplasty is based on polymethyl methacrylate.

In US 2005/0209695 A1 an elastic form stable material is disclosed as an improvement over polymethyl methacrylate based vertebroplasty compositions. Preferred materials include medical grade silicone elastomers. Specifically hydroxyl terminated poly (dimethyl) siloxane is disclosed as matrix polymer, that can be crosslinked by propyl orthosilicate using tin (II) octoate as an initiator. Crosslinking occurs with splitting of propanol. The composition may comprise a radiopaque material to allow X-Ray guidance, such as lateral projection fluoroscopy, during injection. Silver powder is said to be preferred as opacifier agent because of its additional antibacterial and anti-microbial activity.

Despite its beneficial properties the elastic form stable material according to US 2005/0209695 A1 some drawbacks have been discovered. One drawback concerns the dosing ratio of polymer : catalyst, which is in the order of 1000 : 1. As the bone filler material is prepared shortly in advance of the actual injection, otherwise (partial) curing has rendered the material unworkable, in an appropriate amount typically in the ml range, the dosing amount of the catalyst is in the microliter range. Any unintended deviation affects the actual curing time and other properties, which is unpredictable and therefore undesired. Thus precise control of the crosslinking reaction during treatment is difficult to achieve. Another drawback relates to the fact that some kind of shrink occurs during crosslinking in the vertebral body. This shrink could cause voids to be present in the crosslinked material, thereby deteriorating the resulting mechanical properties. Furthermore it has appeared that the crosslinked material is susceptible to degradation over time in the presence of e.g. oxygen, resulting in gradually increasing brittleness. If the material becomes too brittle, fractures could be initiated, thereby decreasing its strength. There is also the risk that fine pieces broken away from the bone filler material migrate to other parts of the body. The known material also requires storage at low temperatures (-18°C) in a freezer in a protective atmosphere.

Therefore, it is an object of the present invention to provide a bone filler composition and use thereof in a vertebral body, which composition can be easily prepared and delivered to a vertebral body.

Another object of the present invention is to provide a bone filler composition and use thereof in a vertebral body, which composition after curing is stable in time.

A further object of the present invention is to provide a bone filler composition and use thereof in a vertebral body, wherein one or more of the drawbacks mentioned above are reduced or eliminated.

### SUMMARY OF THE INVENTION

In a first aspect the present invention provides a vertebroplasty method for treating a disordered vertebral body in a living creature, in particular a human being, comprising the step of injecting a curable bone filler composition in said vertebral body, wherein the curable bone filler composition comprises an addition curable polysiloxane system.

In a second aspect the present invention provides a prepackaged addition curable polysiloxane system device for treating a disordered vertebral body in a living creature, in particular a human being, comprising a two component injection device having two containers separated from each other by a temporary seal.

The present inventors have discovered that many of the drawbacks described above relate to the curing mechanism of the polysiloxanes. The composition disclosed in US 2005/0209695 A1 is crosslinked through a so called condensation cure mechanism, wherein upon crosslinking propanol is splitted of. This is believed to be a main cause of the shrink of the known material in the vertebral body. Contrary to a condensation cure mechanism, a bone filler composition according to the invention is crosslinked by means of an addition cure mechanism, wherein the polymer base, a polysiloxane, reacts with a crosslinker without generating a byproduct. Such a byproduct is considered to be responsible for shrink and degradation over time. In addition, addition curable polysiloxane compositions according to the invention can be prepared in a 1:1 ratio from a pre-filled, two container mixing and dispensing system. In such a system almost equal volumes of the starting components can be maintained separately until needed.

The general treatment steps for performing the invention are disclosed in US 2005/0209695, including pre-examination, anaesthesia and surgery.

According to the invention in a creature such as a human being that is in need of a vertebroplasty treatment, an effective amount of the addition curable polysiloxane system into the vertebral body by means of a suitable injection device. The starting materials of this system do not need to be stored at low temperatures. Usually the addition curable polysiloxane system can be processed up to about 10-20 minutes, e.g. 15 minutes. Thereafter curing has advanced to such an extent that the viscosity becomes too high for injection. Injection is usually monitored by X-ray imaging. Once cured, the addition cured polysiloxane bone filler composition provides reinforcement and strength to the treated vertebral body. The cured bone filler composition is less susceptible to breakdown over time, as a result stability is longlasting.

It is also contemplated that the addition curable polysiloxane system is beneficial for filling other areas or sites in need of filling, reinforcement or support for a bone or joint, e.g. due to an injury or disease, excluding cartilage.

Here it is noted that WO 2007/062082 has disclosed a method and composition of repair and reconstruction of intervertebral discs and other reconstructive surgery using an elastic form stable material. The only example presented is a mixture comprising vinyldimethyl terminated dimethyl polysiloxane and trimethylsiloxy terminated polydimethyl siloxane, which polymers are crosslinked by trimethyl methylhydro dimethyl siloxane using a Pt catalyst.

Thus, the invention also comprises a method for treating a bone structure except disorders of the intervertebral disc of a living creature, comprising the step of contacting the bone structure with an addition curable polysiloxane composition comprises an ethylenically unsaturated moiety terminated polysiloxane, a crosslinker and a catalyst in effective amounts for allowing an addition curing *in situ.*

### DETAILED DESCRIPTION OF THE PREFERRED EMBODIMENTS

The bone filler composition injected according to the invention cures *in situ* in the vertebral body by means of a so called addition cure mechanism. Basically this mechanism involves the addition reaction of polyfunctional silicon hydride to unsaturated groups in polysiloxane chains. The E-modulus of the cured material is higher than the E-modulus of the known polysiloxane based materials. Nevertheless the cured material maintains a certain degree of flexibility due to relatively long chains. This balance of properties results in a reduced risk of fracture. And even, if a fracture occurs, migration is less likely.

In a preferred embodiment the addition curable polysiloxane system comprises a polysiloxane having ethylenically unsaturated moieties. The ethylenically unsaturated moiety or moieties can be present on an end Si, but also as a substituent to the polysiloxane. Vinyl and unsaturated aromatics such as phenyl are preferred examples of these moieties. Preferably an ethylenically unsaturated moiety terminated polysiloxane, more preferably a vinyl terminated polysiloxane such as vinyldialkyl terminated dimethyl polysiloxane, even more preferably a vinyldimethyl terminated dimethyl polysiloxane is used in the addition curable polysiloxane system. The above polysiloxanes are commercially available from several manufacturers including Nusil, Dow Corning and UCT.

The addition curable polysiloxane system also comprises a catalyst, in particular a precious metal catalyst, preferably Pt. More preferably this catalyst is present as an organic Pt(0) complex such as a Pt(0) vinyl siloxane complex.

The addition curable polysiloxane system also requires a crosslinker for addition cure of the base polymeric polysiloxanes described above. Typically the crosslinker will be a hydrosilicone crosslinker having a number of hydrogen atoms capable of reaction with the ethylenically unsaturated groups of the base polymer(s). Preferably, the crosslinker comprises hydrogendialkyll terminated siloxy groups, preferably hydrogendimethyl terminated siloxy groups. Another preferred type of crosslinker is polyalkyl hydrosiloxanes wherein hydrogen is present in each unit of the siloxane chain. Polymethylhydrosiloxanes are an example. The crosslinker will hydrosilylate olefins in the presence of precious metal catalysts, such as Pt.

A system according to the invention is typically such that the viscosity thereof allows extrusion through a needle, e.g. an 11 gauge needle, and such that easy injection into a vertebral disk is possible. After *in vivo* curing the material obtained is sufficiently thixotropic which effectively prevents leakage from the vertebral disk concerned. Curing takes place at low temperature. During *in vivo* curing the local temperature will not raise higher than 40°C, in particular not higher than body temperature, so that no further damage and necrosis of the bone occurs. Typically the addition curable polysiloxane system also comprises a radiopaque material in order to monitor the progress of the vertebroplasty treatment of the invention. Suitable radiopaque materials include silver powder, barium sulphate, bismuth trioxide, zirconium dioxide, tantalum or titanium powders or fibers, calcium sulphated, calcium phosphate, hydroxyapetite, tri calcium phosphate, and other medically appropriate opacifier agents. A preferred radiopaque material is titanium dioxide because of its bone (in)growth enhancing properties, which would promote adhesion between the bony structure of the vertebral body and the addition cured polysiloxane.

In a further preferred embodiment a bactericidal agent such as silver powder is also present in the bone filler composition. Silver powder is an example thereof. Preferably the bactericidal agent comprises a Ag ⁺ zeolite, especially a Ag ⁺ nano zeolite. Such a zeolite allows ion exchange, e.g. substitution of silver ions by sodium ions from the body. This is a relatively slow process thereby effectively extending the time period during which Ag ions are released. Furthermore this bactericidal agent prevents the formation of a so called biofilm affecting adhesion and promoting inflammation risk.

A filler like silicate could also be present in the addition curable polysiloxane system according to the invention.

The composition is advantageously packaged as a kit of parts, comprising at least a first container with a catalyst, and a second container with the cross-linking agent, wherein advantageously the base polysiloxane polymer(s) is present in both containers.

In another preferred embodiment the addition curable polysiloxane system is provided as a two component system, comprising
as part A:
- a vinyl terminated polysiloxane,
- a catalyst
- a radiopaque material a bactericidal agent
- optionally a filler
and as part B;
- a vinyl terminated polysiloxane
- a crosslinker
- a radiopaque material
- a bactericidal agent
- optionally a filler.

An example of a suitable two component system including a mixing functionality is described in PCT/NL2004/000827 and PCT/NL2005/000268. The preferred, advantageous and/or beneficial embodiments of the various components of the composition similarly apply to this embodiment. In particular, the composition is packaged in a two component mixing and injection device having two containers temporarily sealed from each other, wherein part A is present in a first container and part B is present in a second container, and wherein one container is provided with an actuable stirring mechanism. Usually the device will also comprise a detachable needle. Advantageously the addition curable polysiloxane is injected in an effective amount for achieving a shore A durometer > 40, preferably > 60, in particular Shore D durometer > 50 (i.e. Shore A durometer > 90). It has been found that the durometer of the vertebroplasty material known from US 2005/0209695 is low, too soft for high loads on the vertebral body treated. The invention also relates to a prepackaged addition curable polysiloxane system for treating a disordered vertebral body in a living creature, in particular a human being, comprising a two component injection device having two containers separated from each other by a temporary seal, wherein a first container comprises
- a vinyl terminated polysiloxane,
- a catalyst
- a radiopaque material
   a bactericidal agent
- optionally a filler
and a second container comprises;
- a vinyl terminated polysiloxane
- a crosslinker
- a radiopaque material
- a bactericidal agent
- optionally a filler.

The preferred, advantageous and/or beneficial embodiments of the various components of the composition similarly apply to this device.

A further aspect of the invention is the use of an addition curable polysiloxane system in the manufacture of a bone filler composition for treating a disordered vertebral body in a creature, in particular a human being.

## Claims

1. Use of an addition curable polysiloxane system in the manufacture of a bone filler composition for use in a vertebroplasty method for treating a disordered vertebral body in a creature.

2. Use according to claim 1, wherein said addition curable polysiloxane system comprises a polysiloxane having ethylenically unsaturated moiety.

3. Use according to claim 1, wherein said addition curable polysiloxane system comprises a vinyl terminated polysiloxane.

4. Use according to claim 2, wherein said polysiloxane having ethylenically unsaturated moiety comprises a vinyldialkyl terminated dimethyl polysiloxane.

5. Use according to claim 1, wherein the addition curable polysiloxane system comprises a Pt catalyst.

6. Use according to claim 5, wherein the Pt catalyst comprises a Pt(0) vinyl siloxane complex.

7. Use according to claim 1, wherein the addition curable polysiloxane system comprises a hydrosilicone crosslinker.

8. Use according to claim 7, wherein the hydrosilicone crosslinker comprises hydrogendialkyl terminated siloxy groups.

9. Use according to claim 1, wherein the addition curable polysiloxane system further comprises a radiopaque material.

10. Use according to claim 1, wherein the addition curable polysiloxane system comprises Ag⁺ zeolite as bactericidal agent.

11. Use according to claim 1, wherein the addition curable polysiloxane system is provided as a two component system, comprising
as part A:
- a vinyl terminated polysiloxane,
- a catalyst
- a radiopaque material
a bactericidal agent
- optionally a filler
and as part B;
- a vinyl terminated polysiloxane
- a crosslinker
- a radiopaque material
- a bactericidal agent
- optionally a filler.

12. Use according to claim 11, wherein said composition is packaged in a two component injection device having two containers temporarily sealed from each other, wherein part A is present in a first container and part B is present in a second container.

13. Use according to claim 11, wherein said composition is packaged in a two component mixing and injection device having two containers temporarily sealed from each other, wherein part A is present in a first container and part B is present in a second container, and wherein one container is provided with an actuable stirring mechanism.

14. Prepackaged addition curable polysiloxane system for use in a vertebroplasty method for treating a disordered vertebral body in a living creature, in particular a human being, comprising a two component injection device having two containers separated from each other by a temporary seal, wherein a first container comprises
- a vinyl terminated polysiloxane,
- a catalyst
- a radiopaque material
a bactericidal agent
- optionally a filler
and a second container comprises;
- a vinyl terminated polysiloxane
- a crosslinker
- a radiopaque material
- a bactericidal agent
- optionally a filler.

## Patentansprüche

1. Verwendung eines durch Addition vernetzenden Polysiloxansystems bei der Herstellung einer Knochenfüllstoff-Zusammensetzung zur Verwendung in einem Vertebroplastieverfahren zum Behandeln eines erkrankten Wirbelkörpers in einer Kreatur.

2. Verwendung nach Anspruch 1, bei welcher das durch Addition vernetzende Polysiloxansystem ein Polysiloxan mit einem ethylenisch ungesättigten Teil umfasst.

3. Verwendung nach Anspruch 1, bei welcher das durch Addition vernetzende Polysiloxansystem ein vinylterminiertes Polysiloxan aufweist.

4. Verwendung nach Anspruch 2, bei welchem das Polysiloxan mit einem ethylenisch ungesättigten Teil ein vinyldialkylterminiertes Dimethylpolysiloxan aufweist.

5. Verwendung nach Anspruch 1, bei welcher das durch Addition vernetzende Polysiloxansystem einen Pt-Katalysator aufweist.

6. Verwendung nach Anspruch 5, bei welcher der Pt-Katalysator einen Pt(0) Vinyl-Siloxan-Komplex aufweist.

7. Verwendung nach Anspruch 1, bei welcher das durch Addition vernetzende Polysiloxansystem einen Hydrosilikonvernetzer aufweist.

8. Verwendung nach Anspruch 7, bei welcher der Hydrosilikonvernetzer hydrogendialkylterminierte Siloxy-Gruppen aufweist.

9. Verwendung nach Anspruch 1, bei welcher das durch Addition vernetzende Polysiloxansystem ferner ein radiopakes Material aufweist.

10. Verwendung nach Anspruch 1, bei welcher das durch Addition vernetzende Polysiloxansystem einen Ag⁺-Zeolith als bakterizides Mittel aufweist.

11. Verwendung nach Anspruch 1, bei welcher das durch Addition vernetzende Polysiloxansystem als ein Zweikomponentensystem bereitgestellt wird, mit
als einem Teil A:
- einem vinylterminierten Polysiloxan,
- einem Katalysator
- einem radiopaken Material
- einem bakteriziden Mittel
- optional einem Füllstoff
und als einem Teil B;
- einem vinylterminierten Polysiloxan
- einem Vernetzer
- einem radiopaken Material
- einem bakteriziden Mittel
- optional einem Füllstoff.

12. Verwendung nach Anspruch 11, bei welcher die Zusammensetzung verpackt ist in einer Zweikomponenten-Injektionseinrichtung mit zwei Behältern, die zeitweise gegeneinander abgedichtet sind, wobei Teil A in einem ersten Behälter vorhanden ist und Teil B in einem zweiten Behälter vorhanden ist.

13. Verwendung nach Anspruch 11, bei welchem die Zusammensetzung in einer Zweikomponenten-Misch- und Injektionseinrichtung mit zwei zeitweise gegeneinander abgedichteten Behältern verpackt ist, wobei Teil A in einem ersten Behälter vorhanden ist und Teil B in einem zweiten Behälter vorhanden ist, und wobei ein Behälter mit einem betätigbaren Rührmechanismus versehen ist.

14. Vorverpacktes durch Addition vernetzendes Polysiloxansystem für die Verwendung in einem Vertebroplastieverfahren zum Behandeln eines erkrankten Wirbelkörpers in einer lebenden Kreatur, insbesondere in einem menschlichen Wesen, mit einer Zweikomponenten-Injektionseinrichtung mit zwei voneinander durch eine temporäre Dichtung separierten Behältern, wobei ein erster Behälter umfasst
- ein vinylterminiertes Polysiloxan,
- einen Katalysator
- ein radiopakes Material
- ein bakterizides Mittel
- optional einen Füllstoff
und ein zweiter Behälter umfasst;
- ein vinylterminiertes Polysiloxan
- einen Vernetzer
- ein radiopakes Material
- ein bakterizides Mittel
- optional einen Füllstoff.

## Revendications

1. Utilisation d'un système polysiloxane durcissable par addition dans la fabrication d'une composition de remplissage osseux destinée à être utilisée dans une technique de vertébroplastie pour traiter un corps vertébral fragilisé chez une créature.

2. Utilisation selon la revendication 1, dans laquelle ledit système polysiloxane durcissable par addition comprend un polysiloxane contenant un fragment à insaturation éthylénique.

3. Utilisation selon la revendication 1, dans laquelle ledit système polysiloxane durcissable par addition comprend un polysiloxane à terminaisons vinyle.

4. Utilisation selon la revendication 2, dans laquelle ledit polysiloxane contenant un fragment à insaturation éthylénique comprend un diméthylpolysiloxane à terminaisons vinyldialkyle.

5. Utilisation selon la revendication 1, dans laquelle le système polysiloxane durcissable par addition comprend un catalyseur Pt.

6. Utilisation selon la revendication 5, dans laquelle le catalyseur Pt comprend un complexe Pt(0)-vinylsiloxane.

7. Utilisation selon la revendication 1, dans laquelle le système polysiloxane durcissable par addition comprend un agent de réticulation de type hydrosilicone.

8. Utilisation selon la revendication 7, dans laquelle l'agent de réticulation de type hydrosilicone comprend des groupes siloxy à terminaisons hydrogénodialkyle.

9. Utilisation selon la revendication 1, dans laquelle le système polysiloxane durcissable par addition comprend, en outre, un matériau opaque aux radiations.

10. Utilisation selon la revendication 1, dans laquelle le système polysiloxane durcissable par addition comprend une zéolithe Ag⁺ à titre d'agent bactéricide.

11. Utilisation selon la revendication 1, dans laquelle le système polysiloxane durcissable par addition est proposé sous la forme d'un système à deux composants, comprenant
à titre de partie A :
- un polysiloxane à terminaisons vinyle
- un catalyseur
- un matériau opaque aux radiations
- un agent bactéricide
- éventuellement, une charge
et à titre de partie B :
- un polysiloxane à terminaisons vinyle
- un agent de réticulation
- un matériau opaque aux radiations
- un agent bactéricide
- éventuellement, une charge.

12. Utilisation selon la revendication 11, dans laquelle ladite composition est chargée dans un dispositif d'injection à deux composants comportant deux récipients temporairement hermétiques l'un vis-à-vis de l'autre, ladite partie A étant présente dans un premier récipient et la partie B dans un second.

13. Utilisation selon la revendication 11, dans laquelle ladite composition est chargée dans un dispositif de mélange et d'injection à deux composants comportant deux récipients temporairement hermétiques l'un vis-à-vis de l'autre, ladite partie A étant présente dans un premier récipient et la partie B dans un second, un desdits récipients étant pourvu d'un mécanisme d'agitation actionnable.

14. Système polysiloxane durcissable par addition préchargé, destiné à être utilisé dans une technique de vertébroplastie pour traiter un corps vertébral fragilisé chez une créature vivante, en particulier, l'homme, comprenant un dispositif d'injection à deux composants comportant deux récipients séparés l'un de l'autre par un joint hermétique temporaire, un premier récipient comprenant
- un polysiloxane à terminaisons vinyle
- un catalyseur
- un matériau opaque aux radiations
- un agent bactéricide
- éventuellement, une charge
et un second récipient comprenant :
- un polysiloxane à terminaisons vinyle
- un agent de réticulation
- un matériau opaque aux radiations
- un agent bactéricide
- éventuellement, une charge.
